# EUROPEAN PATENT SPECIFICATION

(11) **EP 1 590 669 B1**
(45) Date of publication and mention of the grant of the patent: **25.11.2009**
(21) Application number: 04708674.9
(22) Date of filing: 05.02.2004
(51) Int. Cl.: G01N 33/537, C12Q 1/52, G01N 33/543, G01N 33/569, G01N 33/58

(54) **A MICROCHIP-BASED SYSTEM FOR HIV DIAGNOSTICS**
SYSTEM AUF MIKROCHIPBASIS ZUR HIV-DIAGNOSTIK
SYSTEME BASE SUR UNE PUCE POUR LE DIAGNOSTIC DU VIH

(30) Priority: 05.02.2003 US 445143 P; 13.02.2003 US 447070 P; 24.07.2003 WO PCT/US03/23131
(43) Date of publication of application: 02.11.2005
(73) Proprietor: THE GENERAL HOSPITAL CORPORATION, Boston, MA 02114 (US); BOARD OF REGENTS, THE UNIVERSITY OF TEXAS SYSTEM, Austin, TX 78701 (US)
(72) Inventor: WALKER, Bruce, D., Boston, MA 02114 (US); RODRIGUEZ, William,, Charleston, MA 02129 (US); MCDEVITT, John, T., Austin, TX 78735 (US); CHRISTODOULIDES, Nick J, Austin, TX 78704 (US)
(74) Representative: W.P. Thompson & Co.
(86) International application number: PCT/US2004/003610
(87) International publication number: WO 2004/072097

(56) References cited:
- WO-A-97/35181
- WO-A2-02/061392
- US-A- 5 674 698
- US-A- 5 756 291
- HUANG C M ET AL: "ENZYME ABNORMALITIES OF PATIENTS WITH ACQUIRED IMMUNODEFICIENCY SYNDROME" CLINICAL CHEMISTRY, vol. 34, no. 12, 1988, pages 2574-2576, XP008079416 ISSN: 0009-9147

## Description

### FIELD OF THE INVENTION

The invention relates to microchip-based assays to measure HIV-associated analytes of interest (e.g., CD4 lymphocytes, HIV RNA and liver enzymes) in subjects infected with the HIV virus. Methods of the present invention are optimal for use in monitoring HIV disease in resource-poor settings.

### BACKGROUND OF THE INVENTION

Human immunodeficiency virus, or HIV, is the cause of acquired immune deficiency syndrome (AIDS), a paramount global health problem. AIDS was first described in the early 1980s, and is characterized by profound immune dysfunction, with diverse clinical features such as opportunistic infections, malignancies, and central nervous system degeneration. Although significant progress has been made in the molecular characterization of the virus and treatment modalities for AIDS-related symptoms, there is still much to be done toward the eradication of HIV in infected patients. To date, no successful vaccines have been produced, and currently available drugs fail to address the issue of HIV's frequent and rapid mutation rates.

HIV is a retrovirus belonging to the lentivirus family. HIV has at least two subtypes: HIV-1 and HIV-2. HIV-1 is the predominant subtype in the United States, while HIV-2 is prevalent in West Africa. Infectious HIV particles consist of two plus-strand RNA molecules, each comprised of a 9.3 kilobase genome. The HIV genome is packaged within a core of viral proteins and is surrounded by a phospholipid membrane bilayer, derived from the host cell. This phospholipid bilayer contains proteins originating from the host cell, in addition to virally encoded membrane proteins. The architecture of the HIV genome is based upon nucleotide sequences referred to as *gag, pol,* and *env*. Gag encodes a polyprotein that is proteolytically processed to yield the core structural proteins of the HIV virion. Gag is the most abundant protein in the virion and comprises nearly 90% of the viral structural proteins. Gag is also the only protein required for infectious particle formation, and as such, assembly of Gag at the plasma membrane of the host cell is the driving force for virion production. Gag encodes the mature protein matrix ("MA"), capsid ("CA"), spacer peptide 1 ("SP1"), nucleocapsid ("NC"), SP1, and p6^{Gag}. The polyprotein is cleaved by a protease encoded by *pol*. Pol sequences encode reverse transcriptase, endonuclease, and viral protease enzymes that are required for replication of the viral genome. Pol is expressed by a -1 frameshift during Gag translation, and produces a Gag-Pol polyprotein that, when processed, results in structural reorganization of the virion after budding. Env sequences encode the glycoproteins gp120 and gp41, which reside on the virion envelope.

Additionally, other genes active in viral particle formation, *vpr, vif, tat, rev, nef,* and *vpu,* are contained in the HIV genome. Tat is a 14 kD protein with transcriptional, post-transcriptional, and translational activities that stimulate expression of all HIV genes. Rev is a 20 kD protein that stabilizes, processes, and transports viral messenger RNA molecules encoding *gag, pol,* and env genes, while simultaneously down-regulating expression of *tat, nef,* and *rev* itself. *Nef* encodes a prenylated protein whose mechanism of action is unknown. Similarly, the proteins encoded by *vpr, vif*, and *vpu* are not well characterized, but may play a role in viral particle infectivity.

There are several differences between HIV-1 and HIV-2. For example, HIV-2 subtypes express an additional gene, *vpx,* and lack the *vpu* gene. Like *vpu*, *vpx* is poorly characterized. Further, the rev gene in HIV-2 contains a large insertion, compared with that in HIV-1. Finally, differences in the *env* genes between HIV-1 and HIV-2 result in differences in antibody recognition. Thus, diagnostic tests for HIV must distinguish between these two viral types. In addition, HIV-1 and HIV-2 both undergo significant mutations in response to pressures from the immune system or from antiretroviral drugs. Diagnostics tests also need to be able to distinguish between the innumerable variety of viral subtypes resulting from these mutations.

HIV primarily infects T-lymphocytes that express the cell-surface antigen CD4, but also infects macrophages and follicular dendritic cells in the lymph node, which serve as antigen-presenting cells. The CD4 protein binds to the gp120 glycoprotein of HIV with high-affinity, which propagates a membrane fusion event, whereby the HIV genome can be transmitted from cell to cell. Membrane fusion and internalization of the virion into the cell is facilitated through the gp41 protein. A plurality of host cellular factors also governs entry of the virion into the cell. Upon entry, enzymes within the nucleoprotein complex are activated and the RNA genome of HIV is reverse transcribed to yield its corresponding DNA molecule, which becomes integrated into the host cell genome by the virally-encoded integrase. Integration is also enhanced by concomitant T-cell activation in response to viral entry, however the provirus can remain transcriptionally inactive for months or even years.

Transcriptional upregulation of the integrated DNA provirus is achieved by two long terminal repeats (*LTRs*) flanking either side of the structural genes. These cis-acting sequences are recognized by host proteins, such as nuclear factor κB (NF-κB) and the transcription factor SP1. As with other critical steps in virion internalization, transcription of viral proteins is facilitated by host proteins and host signal transduction mechanisms. For example, transcription is enhanced by the upregulation of small cellular factors known as cytokines. These signaling molecules modulate immune system function by participating in a variety of cellular events such as, but not limited to, hematopoietic cellular differentiation, cell-surface antigen expression, apoptosis, and antibody antigen recognition. Cytokines include tumor necrosis factor (TNF), the interferons (IFN α, β, and y), and the interleukins. From a different perspective, it can be stated that the same mechanisms that drive proliferation and maintenance of CD4-expressing cells also drive HIV replication.

Antiretroviral therapy has been plagued by an inescapable fact: that HIV undergoes rapid and frequent mutagenesis of its genome. Many treatments are directed to one particular viral protein, and while viral replication and processing can be strongly suppressed by combination therapies, HIV retains the capacity to replicate slowly in several different body compartments. As such, they are able to mutate and eventually circumvent the drug or drugs. The current armament of drugs and treatment regimens are ultimately insufficient for long-term control of viral replication, however there are other targets of the viral life cycle that have not been explored. Two main classes of drugs are currently available: nucleoside inhibitors that target reverse transcriptase, and protease inhibitors, which inhibit proteolytic processing of polyproteins encoded by the viral genome (Menendez-Arias, L. 2002. Trends Pharm. Sci. 23(8): 381-388). A third class of drugs has recently been added to the formulary of FDA-approved drugs, entry inhibitors, which inhibit fusion of the HIV virion with the membrane of CD4-expressing cells.

Inhibitors of HIV reverse transcriptase ("RT") include nucleoside analog inhibitors ("NRTIs", i.e. zidovudine monophosphate), acyclic nucleoside phosphonates (i.e. tenofovir), non-nucleoside RT inhibitors ("NNRTIs", i.e. nevirapine), and pyrophosphate analogs. Nucleoside inhibitors act as competitive inhibitors of HIV-RT substrates, and upon phosphorylation, these drugs act as chain terminators and prevent elongation of the growing DNA chain. Resistance to this class of inhibitors is caused by mutations of residues close to the nucleotide-binding site of HIV-RT, but can also occur through mutations that enhance removal of chain-terminating drugs, such as zidovudine monophosphate ("AZT"), from blocked DNA primers through phosphorolysis, mediated by either ATP or pyrophosphate. While the mutations and amino acids differ among different HIV-RT inhibitors, it is clear that the mechanism of action of all these types of RT inhibitors can be circumvented.

Another target of drug activity is the HIV protease ("PR"). As detailed above, the protease is responsible for proteolytic processing of proteins encoded by the HIV genome. The HIV-PR inhibitors act as competitive inhibitors of the proteolytic reactions. Some examples include indinavir and saquinavir. Primary resistance mutations are generally found in residues contained in the substrate-binding pocket. These mutations reduce catalytic activity of the protease and ultimately, viral replication. However, additional mutations can compensate for proteolytic function of the enzyme. Therapies combining both PR and RT inhibitors have had limited success in controlling viral replication, however, combination therapy with multiple drugs results in a different spectrum of mutations compared to therapy with just one drug or type of drug. Other targets of the viral life cycle are subjects of intense research, such as the gp41 protein and integrase. One drug that targets the gp41/gp120 molecule and prevents fusion is currently available as an entry inhibitor, (enfuvirtide).

There has been recent progress toward bringing effective antiretroviral treatments to the world's poorest countries, but most are devastated by the HIV pandemic. In May 2002, the Global Fund to Fight AIDS, Tuberculosis and Malaria disbursed $616 million to support 58 projects in 37 countries, 70% of which target HIV/AIDS (Global Fund to Fight AIDS, Tuberculosis, and Malaria, Global Fund Update, June 2002). In addition, locally driven efforts, such as Clinique Bon Saveur in central Haiti, have proven that standard-of-care HIV therapy can and should be brought to HIV-infected people in even the most remote regions (Farmer, P.E. 2002. XIVth International AIDS Conference). Nonetheless, only 50,000 of an estimated 25 million people in developing countries who need antiretroviral treatment currently receive it, and only 40,000 more will be covered by Global Fund projects in 2003 (Global Fund to Fight AIDS, Tuberculosis, and Malaria, Global Fund Update, June 2002).

An important issue that has not been addressed by the Global Fund and by most advocates of HIV treatment programs in underdeveloped countries is the lack of affordable HIV laboratory tests necessary to monitor treatment regimens. Implementation of HIV laboratory testing in resource-poor settings faces financial and structural obstacles that in some cases dwarf those facing drug procurement programs. While treatment programs strive to obtain expensive drugs in settings where total annual per capita health expenditures average $45 (Musgrove, P. et al, 2002. Bull. World Health Organ. 80: 134-146), cost estimates for HIV laboratory tests range from $140 to $1,500 per patient per year in developing countries (Floyd, K. and C. Gilks, 1997. World Health Organization; Schwartlander, B. et al, 2001. Science 292: 2434-6). Moreover, current HIV laboratory tests require sophisticated laboratory equipment, trained technical staff, and reliable electricity and refrigeration, all of which are scarce resources in much of the HIV-infected world. If treatment programs in resource-poor settings are to be effective, it is critical that equal attention is paid to the question of how HIV-infected patients worldwide are monitored.

Serologic assays to detect HIV antibodies as a means of diagnosing HIV infection remain the only widely available HIV tests. More than 24 million HIV serologies are performed annually in the United States (Centers for Disease Control and Prevention, Annual Report 1997-1998. June 2001), and untold millions are performed worldwide. In the United States and developed countries, a screening enzyme-linked immunosorbent assay (ELISA) is performed, followed by a confirmatory Western blot if the ELISA is positive. The need for cheaper and more rapid serologic tests in resource-poor settings led to the development of rapid ELISAs, which can be performed for a total cost of $3 to $5 per patient.

While serologic tests to diagnose HIV became standardized in the mid-1980s, monitoring the progression of HIV infection with laboratory tests has proved to be more difficult. Initially, clinical parameters such as weight loss or the development of opportunistic infections were used to stage HIV disease, but failed to identify many patients with advanced AIDS who remained asymptomatic (Murray, H.W. et al, 1989. Am. J. Med. 86: 533-8; Kaplan, J.E. et al, 1992. J. Acquir. Immune Defic. Syndr. 5: 565-70). Once effective interventions with prophylactic antibiotics and antiretroviral therapies became available, useful laboratory markers of HIV disease status were needed.

Several candidate serologic markers were studied in the late 1980s and early 1990s, including HIV p24 antigen (Lange, J.M. et al, 1987. AIDS 1: 155-9), anti-HIV antibodies, serum IgA (immunoglobulin A; Schwartlander, B. et al, 1993. AIDS 7: 813-22), IgG, IgM, β-2 microglobulin (Anderson, R.E. et al, 1990. Arch. Intern. Med. 150: 73-7), neopterin (Bogner, J.R. et al, 1988. Klin. Wochenschr. 66: 1015-8), adenosine deaminase and soluble interleukin-2 receptor (IL-2R) levels (Lange, J.M. et al, 1988. Ann. Med Interne (Paris) 139: 80-3; Fahey, J.L. et al, 1990. N. Engl. J. Med. 322: 166-72; Sabin, C.A. et al, 1994. Br. J. Haematol. 86(2): 366-71; Zabay, J.M. et al, 1995. Acquir. Immun. Defic. Syndr. Hum. Retrovirol. 8: 266-72; Planella, T. et al, 1998. Clin. Chem. Lab. Med. 36: 169-73). All of these markers were found to have some utility in certain settings, but failed to show a direct, quantifiable correlation with advancing disease. The clinical utility of routine measurements of these markers remained unproven, and technical limitations plagued several assays. With the exception of p24 testing, they were not widely adopted in clinical practice.

p24 antigen testing was briefly adopted in some laboratories, as most patients with clinical AIDS-defining criteria had significantly higher serum levels of p24 than asymptomatic patients. However, anti-p24 antibody responses develop in many patients, and p24 can then circulate throughout the body as antibody-bound immune complexes. Unless these immune complexes were acid- or heat-denatured before testing, commercial assays underestimated the levels of p24, thereby limiting their clinical utility (Nadal, D. et al, 1999. J. Infect. Dis. 180: 1089-95). Moreover, many patients with advanced AIDS have negligible p24 levels, even after immune complex disruption, for unknown reasons (Ercoli, L., et al 1995. 11: 1203-7). Because of these problems with p24 measurements, and because the assay is labor-intensive, more reliable tests with direct correlation to disease-notably, CD4 counts and HIV RNA levels-became desirable prognostic markers.

The most consistent predictor of clinical disease in early studies of HIV-1 infection proved to be the CD4+ T cell count. Longitudinal studies revealed a slow but steady drop in CD4 count, at a rate of ~50 CD4+ cells/µL per year, as well as a clear correlation between CD4 counts below 200 cells/µL and death (Fahey, J.L. et al, 1990. N. Engl. J. Med. 322: 166-72). Based on these findings, the Centers for Disease Control and Prevention reclassified its definition of AIDS to include patients whose CD4+ T cell count dropped below 200 cells/µL (Centers for Disease Control MMWR 1992; 41 (RR-17): 1-19), a number which quickly became a totem in HIV clinical laboratories. Additional studies suggested that the absolute CD4 count, the percentage of total lymphocytes that were CD4+ ("CD4 percent"), and the CD4:CD8 ratio were all useful markers of disease progression (Centers for Disease Control and Prevention MMWR 1997; 46: 1-29), especially in infants and children.

In 1995 and 1996, a series of publications by Mellors and others dramatically altered the nature of HIV laboratory testing in the developed world (Loveday, C. and A. Hill. 1995. Lancet 345: 790-1; Mellors, J.W. et al, 1995. Ann. Intern. Med. 122: 573-9; Mellors, J.W. et al, 1997. Science 1996. 272: 1167-70 [Erratum appears in Science 275: 14]; Mellors, J.W. et al, 1997. Ann. Intern. Med. 126: 946-54). The amount of HIV RNA present in serum-the "viral load"-was found to correlate directly and convincingly with clinical disease. Additional studies confirmed that the level of HIV RNA in serum was the single most important predictor of the subsequent course of HIV infection in individual patients (Coombs, R.W. et al, 1996. J. Infect. Dis. 174: 704-12; O'Brien W.A. et al, 1997. Ann. Intern. Med. 126: 939-45; O'Brien, W.A. et al, 1996. N. Engl. J. Med 334: 426-31; Yerly, S. et al. 1998. Arch. Intern. Med. 158: 247-52). Measurement of viral load rapidly became standard-of-care, and the basis for treatment decisions with antiretroviral drugs. By 1996, official HIV treatment guidelines supported the measurement of CD4 counts and HIV RNA levels every three months in HIV-infected patients (Carpenter, C.C.J., et al. 1996. JAMA 276: 146-54; Saag, M.S. et al. 1996. Nat. Med. 2: 625-629).

It was also shown that liver enzyme activities, specifically those of alanine aminotransferase and aspartate aminotransferase, were elevated in patients suffering from AIDS and in individuals who were seropositive for HIV, and that the activities of these liver enzymes served as suitable predictors of disease progression (Huang, C.M. et al. 1988. Clin. Chem. 34(12): 2574-6). Moreover, many of the drugs used to treat HIV, and many of the infections that accompany HIV, cause liver damage. Aminotransferases catalyze the removal of α-amino groups from L-amino acids and, as described above, are found primarily in the liver. These α-amino groups are removed during oxidative degradation and are recycled for use in amino acid biosynthesis or are excreted in the form of urea. Transamination results in the collection of all amino groups from different amino acids in the form of L-glutamate. Glutamate channels the amino groups into either biosynthetic or excretory pathways (i.e., the urea cycle in humans). These two enzymes are important, not only in diagnosis of HIV progression, but also in diagnosis of liver damage, and are regularly monitored in most HIV-infected patients.

Weighed against the sensitivity, specificity and clinical utility of the various HIV diagnostic tests is their cost. Costs for immunoassays for serum proteins, including HIV antibodies, p24 antigen, β-2 microglobulin and others, typically range between $3 and $15 per test; newer, rapid tests may cost as little as $1 each. However, immunoassays must be read on a spectrophotometer, which costs roughly $8,000 per machine and requires a steady electrical supply. Even if reliable prognostic markers measurable by immunoassay are developed, these costs remain prohibitive for use in resource-poor countries.

The standard-of-care tests used to monitor HIV treatment in the United States and Europe are markedly different from those described above. CD4 counts are significantly more expensive than immunoassays, ranging from $20 to $100 per test. They also require a flow cytometer, a laser-equipped machine that typically costs $30,000 to $100,000, and has intensive electricity and maintenance requirements. Three HIV RNA tests to measure viral load are currently approved for use. All are amplification-based (e.g., PCR, bDNA or NASBA), cost more than $100 per test, and require sophisticated laboratory settings and highly skilled personnel. Nucleic acid amplification also requires a thermocycler, which can cost as much as $80,000, and demands reliable electricity and temperature control.

Resource-poor settings devastated by HIV have no capacity financial, structural, or technical to perform regular measurements of CD4 count and HIV viral load using these available techniques. Thus, even if HIV medications were made available at no cost, and hospital-, clinic- or community-based treatment programs were implemented, no broadly applicable system currently exists to identify which patients should receive treatment, or how those receiving treatment should be monitored. In the past year, meetings of laboratory scientists, HIV clinicians, international health policy makers and local health ministers have addressed the pressing need for affordable HIV diagnostic and treatment monitoring tools in the developing world. Three approaches have emerged from these meetings: validation of novel surrogate markers of HIV disease (e.g. hemoglobin level); modification of existing tests and assays to resource-poor settings; and development of new technologies to measure CD4 count, HIV RNA and other tests considered standard-of-care in the developed world.

Efforts to identify novel surrogate markers-clinical criteria, total lymphocyte counts, hemoglobin levels-have met with limited success, echoing the limitations of these approaches in the United States and Europe in the late 1980s. An assay to measure reverse transcriptase activity as a surrogate for viral load has shown some promise, but remains costly and technically challenging. A more effective approach has been to modify the p24 assay to create a cheap, rapid immunoassay for heat-denatured p24 antigen (HDp24) (Ledergerber, B., et al. 2000. J. Infect. Dis. 181: 1280-8; Pascual, A., et al. 2002. J. Clin. Microbiol. 40: 2472-5; World Health Organization Global Programme on AIDS. 1994. *AIDS* 8: WHO1-WHO4). In preliminary studies in resource-poor settings, this assay has a sensitivity of 85% and a specificity of 100%, at a cost of $8, and correlates with HIV RNA levels (Pascual, A. et al. 2002. J. Clin. Microbiol. 40: 2472-5). The HDp24 assay is currently the best candidate immunoassay for immediate implementation for treatment monitoring, but further assay improvements and validation studies are necessary, as cost and technical complexity associated with processing remain concerns.

Attempts to develop an affordable flow cytometer for inexpensive CD4 count enumeration have been similarly met with limited success (World Health Organization Global Programme on AIDS. 1994. *AIDS* 8: WHO1-WHO4; Lyamuya, E.F., et al. 1996. J. Immunol. Methods 195: 103-12; Sherman, G.G. et al. 1999. J. Immunol. Methods. 222: 209-17; Janossy, G., et al. 2002. Cytometry 50: 78-85). An alternative approach to CD4 counting using magnetic bead separation (Dynabead^{®}, Dynal, Oslo, Norway) and manual cell counts has proven to be practical in small-scale studies in central reference laboratories in West Africa (Diagbouga, S. et al, 2002. Oral Presentation #WeOrB1342, XIVth International AIDS Conference). The ability to scale up this method to national treatment programs at the regional and district level, as well as the assay's ultimate cost, remain unknown. Currently, the Dynabead^{®} method (U.S. Patent No. 4,910,148) appears to be the only method for CD4 counting that could be implemented immediately at reasonable cost, but its use remains problematic due to technical requirements of the assay (i.e., the Dynabead^{®} method requires CD4+ T cells to be quantified by antibodies labeled with magnetic microspheres and separated by a magnetic separator). While Dynabead^{®} CD4 counts are available now, their costs likely in the range of $3 to $15 per assay-and technical limitations suggest that the future of HIV laboratory testing in resource-poor settings lies in new technologies.

Other technologies have been identified and actively pursued, and may be available in time. General improvements in immunochromatographic technology have been applied to HIV testing. Several cartridge and dipstick tests are available that measure HIV-1 antibodies for HIV diagnosis in a one-step, lateral-flow format (Ketema, F. et al, 2001. J. Acquir. Immune Defic. Syndr. 27: 63-70). Similar assays to measure not only HIV antibodies but also serum proteins that might serve as surrogate markers of clinical HIV disease are currently under evaluation. Single-tube nucleic amplification assays are in active development for a number of applications, including HIV monitoring in resource-poor settings. These assays continue to require sophisticated techniques, but may reduce the cost of HIV RNA measurements to less than $10 (de Baar, M.P. et al, 2001. J. Clin. Microbiol. 39: 1895-902; Oh, C-Y. Monitoring and Diagnostic Tools for the Management of Antiretroviral Therapy in Resource-Poor Settings, Bethesda, Md., Noveber 11-13, 2001).

Another automated method involving the use of a commercially available device, the Biometric Imagn® 2000 and 4T8® cartridge (O'Gorman, M. 1998 Conference on the Laboratory Science of HIV 97-111), also entails problematic technical requirements. For example, analyte capture is performed by centrifugation and detection requires laser scanning, which produces a peak emission profile that must be further processed.

While a global spotlight has appropriately been placed on the lack of access to HIV medications, the concomitant need for affordable HIV diagnostic technologies remains largely unexamined. As money for antiretroviral treatments becomes available, the fact remains that HIV laboratory tests designed for resource-poor settings are urgently needed. New tests need to account not only for severe cost constraints, but also for the lack of refrigeration, electricity, and technical support in the resource-poor settings where the vast majority of the world's 40 million HIV-infected people live.

As discussed above, the most consistent indicators for the degree of damage to the immune system from HIV and for the risk of clinical progression in AIDS are absolute CD4+ T cell counts (the percentage of total lymphocytes that are CD4+) and the CD4: CD8 ratios (Mellors JW et al., 1995). The significance of these markers for monitoring HIV-1 progression was reinforced by United States guidelines, which suggest that treatment should be initiated in all patients with CD4+ counts less than 350 cells/mm³, and that CD4+ counts should be assayed every 3 to 6 months during treatment.

Efforts over the past decade to develop appropriate CD4+ counting methods for resource-poor settings have been unsuccessful. The lack of success is due, in part, to the requirement of conventional assays for sophisticated and expensive equipment, reliable electricity and advanced technical skill. Specifically, an affordable, sensitive, reliable, electricity-independent microchip-based assay for monitoring HIV infection would be highly desirable.

International patent application PCT/US02/03275, published as WO 02/061392 in the name of Board of Regents, The University of Texas System, was cited as D1 during prosecution of this case and concerns a system for characterisation of multi-analyte fluids. It suggests that blood and saliva may be tested for HIV antibodies by attachment of antigens and by use of a sensor array system. In some embodiments the signals detected by the sensor array system are produced by fluorescence.

International patent application PCT/US97/04377, published as WO 97/35181 in the name of University of UTAH Research Foundation, was cited as D2 and describes a system for determining analyte concentration having a CCD or photodetector for detecting fluorescence from fluorescent binding.

### SUMMARY OF THE INVENTION

According to the present invention,there is a method for detecting an HIV-associated analyte in a sample of blood comprising:
a) passing an HIV-associated analyte coupled to a fluorescence-emitting conjugate through a flow cell comprising one or more cavities, wherein one or more of the cavities contains an agarose bead comprising a suitable biological agent having binding affinity for the analyte, such that the analyte is immobilized on the bead, thereby separating the analyte from the blood, wherein the analyte is alanine aminotransferase or aspartate aminotransferase;
b) delivering light to the analyte at a wavelength suitable for excitation of the conjugate;
c) digitally imaging a conjugate emitted fluorescent signal using a detector.

Methods for microchip-based screening of HIV analytes are described herein. Methods of the present invention may utilize an improved microchip flow cell to capture HIV-associated "analytes of interest", such as cells (e.g., CD4 cells), nucleic acids (e.g., HIV RNA) and proteins (e.g., liver enzymes), directly from whole blood. Captured analytes may be imaged using an enhanced fluorescence detection system which combines a system for static imaging, such as a CCD digital camera, with stable fluorescence signaling. Accurate quantification of analytes can be obtained from fingerstick samples of whole blood without the need for further processing or sample amplification.

Captured analytes may be imaged using a light source, one or more dichroic filters, and a detector capable of imaging the immobilized analytes (i.e., "static imaging"). Thus, methods of the present invention provide for efficient capture and static imaging of small molecule analytes of interest.

In one embodiment, CCD digital imaging is used for static imaging. Detection of small molecules may be enhanced through a combination of efficient capture, static imaging and stable fluorescence signaling. Because the small molecules are readily captured and detected, blood sample volume, reagent and power requirements may be significantly reduced. Flow cytometry, by contrast depends upon capture of signals from analytes moving through a flowing stream, thereby requiring more cumbersome processing methods and detection equipment.

Other aspects of the invention are described in or are obvious from (and within the ambit of the invention) the following disclosure.

### BRIEF DESCRIPTION OF THE DRAWINGS

The following Detailed Description, given by way of example, but not intended to limit the invention to specific embodiments described, may be understood in conjunction with the accompanying drawings, incorporated herein by reference, in which:
Figure 1 depicts an expanded view of a membrane based flow sensor.
Figure 2 depicts an embodiment of a membrane based flow sensor disposed in a housing.
Figure 3 depicts a schematic view of an analyte detection system in flow-through mode.
Figure 4 depicts a schematic view of an analyte detection system in lateral flow mode.
Figure 5 depicts a schematic view of an analyte detection system in back-flush mode.
Figure 6 depicts a flow chart of a method of collecting samples.
Figure 7 depicts a 100 microwell microchip, which is slightly smaller than a postage stamp.
Figure 8 depicts a schematic cross-section of the microchip platform. The sample is injected at one end of the microchip and spreads uniformly through the reaction wells, which have a 30 nL volume. Sample drains allow for washing within up to 10,000 dead volumes using only 2 mL of wash buffer. At least two types of microchips can be utilized, one of which contains a polycarbonate membrane on the floor of the microwell for capturing lymphocytes (i.e., for CD4 cell counting) and one of which contains a derivatized agarose microbead in each microwell (for HIV RNA or liver enzyme measurement).

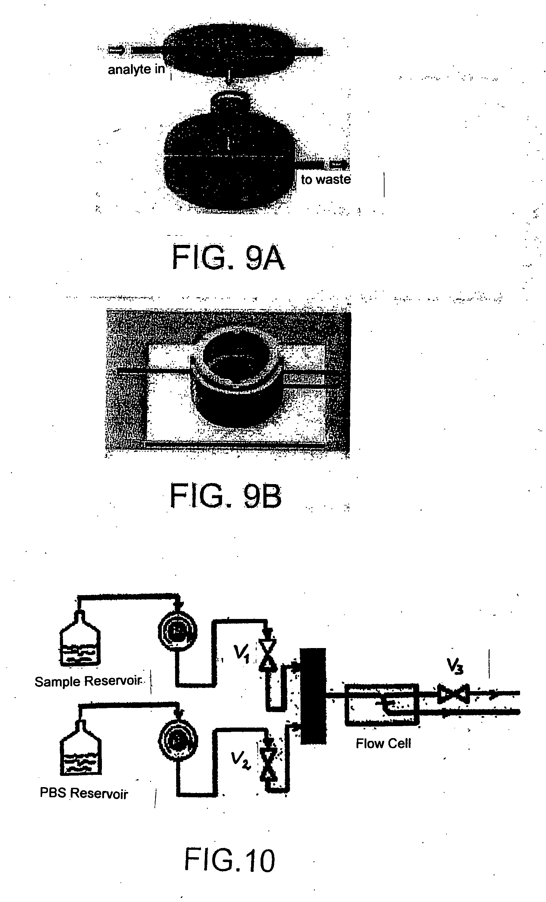

Figures 9a, 9b and 10 depict the experimental design of the flow cell and optical imaging system. Sample is introduced to the flow cell by means of a volumetric mini-pump controlled by a computer (Figure 9A). Cells or analytes are retained in the microwells (Figure 9B), while unretained sample flows through the microwells and into a waste reservoir (Figure 10). A light source (e.g., a mercury pressure lamp) delivers light at an appropriate wavelength or range of wavelengths to the analytes captured in the flow cell microwell at the appropriate focal plane, by means of a dichroic mirror. Fluorescence emitted by the reaction is triggered when excitatory light hits the captured analyte and its fluorescence-emitting detector conjugate and is captured by a CCD digital camera The image can then be stored for analysis.
Figure 11 depicts CD4 lymphocyte dose response. Purified CD4 cells were labeled with Alexa4-88-conjugated anti-CD4 antibodies and introduced to the flow cell in amounts ranging from 0 to 200,000 cells and imaged. There is a linear correlation between the concentration of CD4 cells in the sample and the intensity of light emitted by these labeled lymphocytes captured on the membrane filter (R²= 0.999).

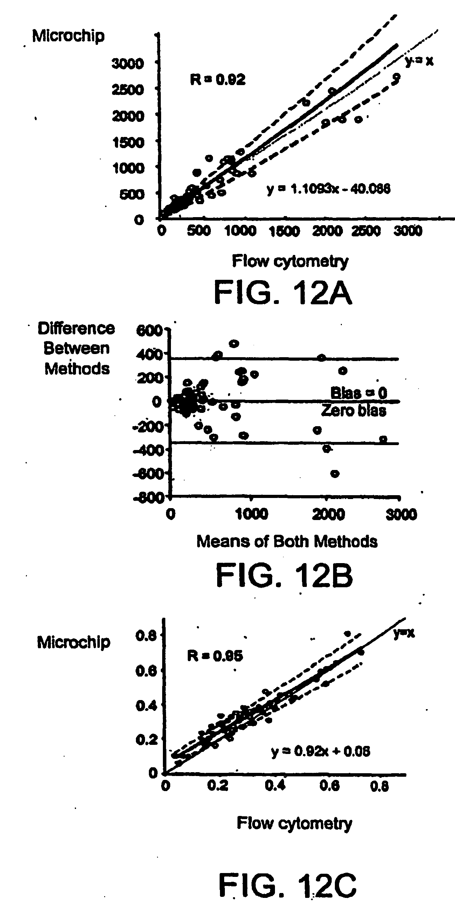

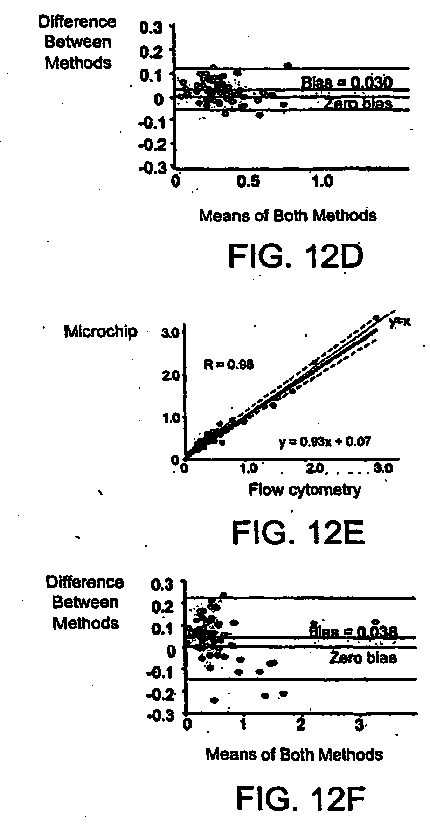

Figure 12 depicts correlation studies comparing a microchip-based system of the present invention with flow cytometry. Agreement between flow cytometry and the novel microchip method is excellent across the range of absolute CD4 counts, CD4 percentages of total T lymphocytes, and CD4/CD8 ratios seen in HIV infection. For absolute CD4 counts, a methods comparison conducted according to the approach of Passing and Bablok showed an excellent correlation with flow cytometry (Figure 12A), and a Bland-Altman plot 24 showed zero bias and excellent 95% limits of agreement (Figure 12B). The calculated Pearson correlation coefficient is 1=0.92. Similar results were obtained for both CD4 counts reported as a percentage of total CD3+ T lymphocytes (Figure 12C-D), and CD4:CD8 ratios (Figure 12E-F).
Figure 13 depicts the results of a microbead experiment. Human serum with anti-HBsAg, and anti-gp41/120 antibodies (at 1:200) was incubated over antigen-coated microbeads and detected with a fluorescent goat anti-human IgG antibody (at 1:200).

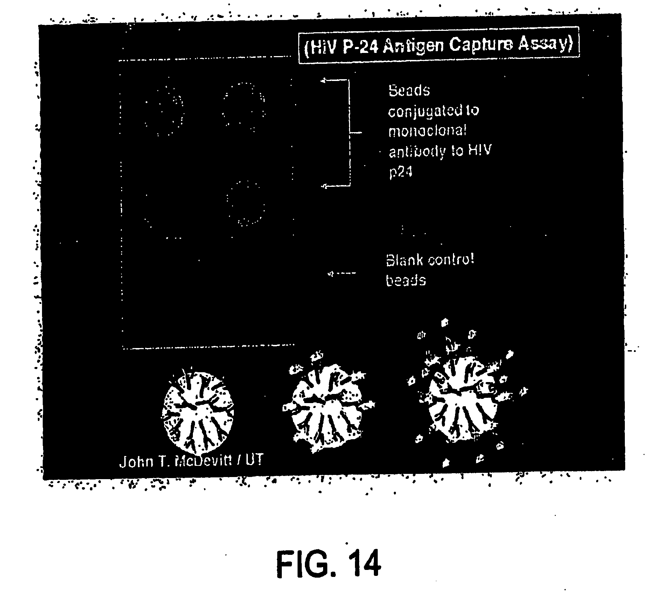

Figure 14 depicts the results of the HIV-p24-antigen microbead assay. Beads coated with antibody to HIV p24 were placed in 4 microwells of a silicon microchip, and reacted with human serum containing 100 pg/mL of HIV p24 antigen. A Cy2-labeled anti-p24 secondary antibody was then added and detected by fluorescence microscopy.
Figure 15 depicts an HIV RNA assay. HIV-specific molecular beacons (clade B gag) were attached to microbeads, and samples containing complementary RNA sequences were introduced to the microchip. Annealing of HIV RNA sequences in the sample with the capture sequence attached to the microchip opens up the molecular beacon, resulting in bright fluorescence. Figure 15A depicts the baseline fluorescent signal. Figure 15B depicts the fluorescent signal after addition of a sample containing 100 pM complementary HIV RNA sequence.
Figure 16 depicts the results of DNA base pair discrimination experiments. Beads were coated with 18 base pair DNA oligomers differing from each other by a single nucleotide. Complimentary sequences for each of the target probes were labeled with distinct fluorochromes, and mixed in a 1:1:1 ratio in solution. Chips were then tested against this equimolar solution, and binding detected by fluorescence microscopy. Fluorochrome-labeled sequences bound only to their complimentary target probes, and not to target probes mismatched by a single nucleotide.

### DETAILED DESCRIPTION OF THE INVENTION

Herein we describe a system and method for the analysis of a fluid containing one or more analytes of interest. As used herein, "analyte" refers to a biological agent that can be efficiently immobilized, detected and quantified using this system, including nucleic acids, proteins, and cells. In one embodiment, analytes of the present invention are associated with HIV infection (i.e., quantification of analytes indicates the presence, or severity, of HIV infection in a subject).

The system may be used for either liquid or gaseous fluids. The system, in some embodiments, may generate patterns that are diagnostic for both the individual analytes and mixtures of the analytes. The system in some embodiments, is made of a plurality of chemically sensitive particles, formed in an ordered array, capable of simultaneously detecting many different kinds of analytes rapidly. An aspect of the system is that the array may be formed using a microfabrication process, thus allowing the system to be manufactured in an inexpensive manner.

In one embodiment of a system for detecting analytes, the system, in some embodiments, includes a light source, a flow cell comprising a sensor array, and a detector. The sensor array, in some embodiments, is formed of a supporting member which is configured to hold a variety of capture agents, such as chemically sensitive particles (herein referred to as "particles") in an ordered array. Examples of particles include, but are not limited to functionalized polymeric beads, agarous beads, dextrose beads, polyacrylamide beads, control pore glass beads, metal oxides particles (e.g., silicon dioxide (SiO₂) or aluminum oxides (Al₂O₃)), polymer thin films, metal quantum particles (e.g., silver, gold, platinum, etc.), and semiconductor quantum particles (e.g., Si, Ge, GaAs, etc.).

A detector (e.g., a charge-coupled device "CCD," a photodiode, a CMOS imager or other light-sensitive device) may be positioned below the sensor array to allow for the data acquisition. In another embodiment, the detector may be positioned above the sensor array to allow for data acquisition from reflectance of the light off of the particles or other capture agents.

Light originating from the light source may pass through the sensor array and out through the bottom side of the sensor array. Light modulated by the particles may pass through the sensor array and onto the proximally spaced detector. Evaluation of the optical changes may be completed by visual inspection or by use of a detector by itself or in combination with an optical microscope. A microprocessor may be coupled to the detector or the microscope. A fluid delivery system may be coupled to the supporting member of the sensor array. The fluid delivery system, in some embodiments, is configured to introduce samples into and out of the sensor array.

A particle, in some embodiments, possess both the ability to bind the analyte of interest and to create a modulated signal. The particles are, in some embodiments, elements that will create a detectable signal in the presence of an analyte. The particles may produce optical (e.g., absorbance or reflectance) or fluorescence/phosphorescent signals upon exposure to an analyte. The particle can be associated with biological agents which posses the ability to bind the analyte of interest and to create a modulated signal. The biological agents can be coupled to indicators (e.g., complementary HIV sequences labeled with fluorescence-emitting compounds). The biological agent may posses the ability to bind to an analyte of interest. Upon binding the analyte of interest, the biological agent may cause the indicator to produce the modulated signal when illuminated. The biological agents may be naturally occurring or synthetic, formed by rational design or combinatorial methods. Some examples include, but are not limited to, DNA, RNA, proteins, enzymes, oligopeptides, antigens, and antibodies. Either natural or synthetic biological agents may be chosen for their ability to bind to the analytes in a specific manner.

In one embodiment, a biological agent is coupled to a light-emitting compound, such as a fluorophore. In another embodiment, the analyte itself can be coupled to the light-emitting compound. These conjugated entities are referred to herein as "fluorescence-emitting detector conjugates".

In one embodiment, the sensor array system includes an array of particles, wherein the particles may comprise a biological agent (e.g., receptor, antibody, DNA or RNA sequence) coupled to one or more polymeric or agarose beads. The biological agents, in some embodiments, are chosen for their ability to interact with analytes. This interaction may take the form of a binding/association, for example, with the analytes. A supporting member may be made of any material capable of supporting the particles, while allowing the passage of the appropriate wavelengths of light.

In one embodiment, a biological agent and an indicator may be coupled to a polymeric resin. Upon capture, a conformational change may take place in the presence of an analyte such that a change in the local microenvironment of the indicator occurs. This change may alter the spectroscopic properties of the indicator. The interaction with the indicator may be produce a variety of different signals depending on the signaling protocol used. Such protocols may include absorbance, fluorescence resonance energy transfer, and/or fluorescence quenching.

In one embodiment, the sensor array system includes an array of particles contained in individual cavities. The particles may include a biological agent coupled to a polymeric or agarose bead. Suitable biological agents can be chosen for interacting with analytes of interest. This interaction may take the form of a binding/association with the analytes. The supporting member may be made of any material capable of supporting the particles, while allowing the passage of the appropriate wavelengths of light. The supporting member may include a plurality of cavities. The cavities may be formed such that at least one particle is substantially contained within the cavity.

The supporting member may include a plurality of cavities, which are also referred to herein as "microwells." Cavities can be formed by micromachining techniques known in the art. The cavities may be formed, for example in pyramidal shaped pits, such that at least one capture agent is contained within the cavity. In one embodiment, the capture agent is a particle comprising an agarose bead or a filter, each of which can be coupled to a biological agent.

The sensor array may include a cover layer. A cover layer may be positioned at a distance above the surface of the sensor array, such that a channel is formed between the sensor array surface and the cover layer. The cover layer may be placed at a distance such that the cover layer inhibits dislodgement of the particles from the cavities in the sensor array, while allow fluid to enter the cavities through the channel formed between the sensor array and the cover layer.

In some embodiments, the cavities may be configured to allow fluid to pass through the cavity during use, while the cavity is configured to retain the capture agent in the cavity as the fluid passes through the cavity. For example, the cavity may comprise an agarose bead coupled to a biological agent having a specific binding affinity for an HIV analyte of interest (e.g., HIV gp41and/orgp120 antigens, HIVp24 antigens and hepatitis B surface antigens).

A vacuum may be coupled to the cavities. The vacuum may be applied to the entire sensor array. Alternatively, a vacuum apparatus may be coupled to the cavities to provide a vacuum to the cavities. A vacuum apparatus is any device capable of creating a pressure differential to cause fluid movement. The vacuum apparatus may apply a pulling force to any fluids within the cavity. The vacuum apparatus may pull the fluid through the cavity. Examples of vacuum apparatus include pre-sealed vacuum chamber, vacuum pumps, vacuum lines, or aspirator-type pumps.

In an embodiment, the optical detector may be integrated within the bottom of the supporting member, rather than using a separate detecting device. The optical detectors may be coupled to a microprocessor to allow evaluation of fluids without the use of separate detecting components. Additionally, a fluid delivery system may also be incorporated into the supporting member. Integration of detectors and a fluid delivery system into the supporting member may allow the formation of a compact and portable analyte sensing system.

A high sensitivity sensor array (e.g., CCD or CMOS) may be used to measure changes in optical characteristics which occur upon binding of the biological/chemical agents. The arrays may be interfaced with filters, light sources, fluid delivery and micromachined particle receptacles, so as to create a functional sensor array. In one embodiment, data acquisition and handling may be performed with existing CCD or CMOS technology. CCD or CMOS detectors may be configured to measure white light, ultraviolet light or fluorescence. Other detectors such as photomultiplier tubes, charge induction devices, photo diodes, photodiode arrays, and microchannel members may also be used.

In one embodiment, a naturally occurring or synthetic biological agent is bound to a polymeric or agarose bead in order to create the particle. The particle, in some embodiments, is capable of both binding the analyte(s) of interest and creating a detectable signal. In some embodiments, the particle will create an optical signal when bound to an analyte of interest.

A variety of natural and synthetic biological agents may be used including, but not limited to, polynucleotides (e.g., aptamers), peptides (e.g., enzymes and antibodies), and receptors. Polynucleotides are relatively small fragments of DNA which may be derived by sequentially building the DNA sequence. Peptides include natural peptides such as antibodies or enzymes or may be synthesized from amino acids.

In one embodiment, the particle may comprise unnatural biopolymers, chemical structure, that are based on natural biopolymers, but which are built from unnatural linking units. For example, polythioureas and polyguanidiniums have a structure similar to peptides, but may be synthesized from diamines (i.e., compounds which include at least two amine functional groups) rather than amino acids.

In one embodiment, a biological agent may be coupled to a polymeric resin. Upon capture, a chemical reaction may take place in the presence of an analyte such that a signal is produced. Indicators may be coupled to the biological agent or the polymeric bead. The chemical reaction may cause a change in the local microenvironment of the indicator to alter the spectroscopic properties of the indicator. This signal may be produced using a variety of signaling protocols. Such protocols may include absorbance, fluorescence resonance energy transfer, and/or fluorescence quenching. Exemplary combinations include, but are not limited to, peptides-proteases, polynucleotides-nucleases, and oligosaccharides-oligosaccharide cleaving agents.

Further details regarding these systems can be found in the following U.S. patent applications: U.S. Patent Application Serial No. 09/287,248 entitled "Fluid Based Analysis of Multiple Analytes by a Sensor Array"; U.S. Patent Application Serial No. 09/354,882 entitled "Sensor Arrays for the Measurement and Identification of Multiple Analytes in Solutions"; U.S. Patent Application Serial No. 09/616,355 entitled "Detection System Based on an Analyte Reactive Particle"; U.S. Patent Application Serial No. 09/616,482 entitled "General Signaling Protocols for Chemical Receptors in Immobilized Matrices"; U.S. Patent Application Serial No. 09/616,731 entitled "Method and Apparatus for the Delivery of Samples to a Chemical Sensor Array"; U.S. Patent Application Serial No. 09/775,342 entitled "Magnetic-Based Placement and Retention of Sensor Elements in a Sensor Array"; U.S. Patent Application Serial No. 09/775,340 entitled "Method and System for Collecting and Transmitting Chemical Information"; U.S. Patent Application Serial No. 09/775,344 entitled "System and Method for the Analysis of Bodily Fluids"; U.S. Patent Application Serial No. 09/775,353 entitled "Method of Preparing a Sensor Array"; U.S. Patent Application Serial No. 09/775,048 entitled "System for Transferring Fluid Samples Through A Sensor Array" (Published as U.S. Publication No.: 2002-0045272-A1); U.S. Patent Application Serial No. 09/775,343 entitled "Portable Sensor Array System"; and U.S. Patent Application Serial No. 10/072,800 entitled "Method and Apparatus for the Confinement of Materials in a Micromachined Chemical Sensor Array".

In another embodiment, the capture agent comprises a membrane based flow sensor which is configured to such that the cavities within the fluidics device contain a filter placed within the fluidics device. Analytes, particularly cells, including lymphocytes and other white blood cells, whose size is larger than the pores of the filter, are captured in the flow cell and immobilized on the membrane. The captured analytes may be analyzed directly or may be treated with visualization compounds priorto imaging.

A variety of analytes may be captured and analyzed using a membrane based flow sensor as described herein. Some analytes that are of particular interested for detection include lymphocytes, monocytes, and other cells of the immune system.

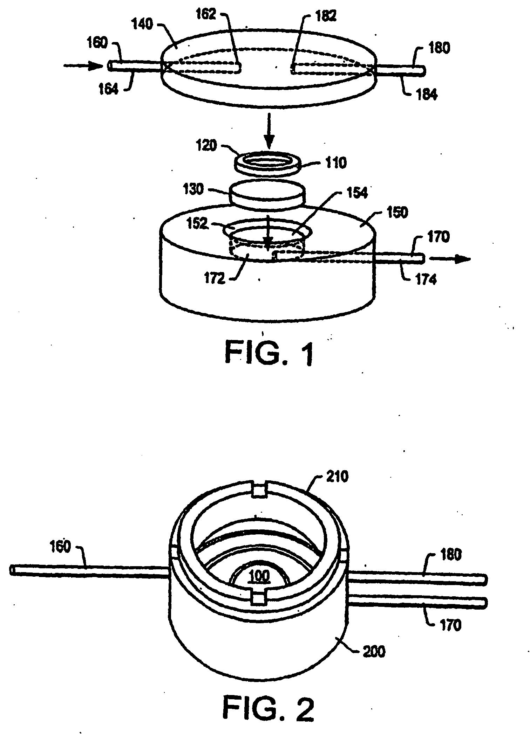

Shown in Figure 1 is an expanded view of a membrane based flow sensor 100. Flow sensor 100 includes a membrane 110 that is sandwiched between at least two members 140 and 150. Members 140 and 150 are configured to allow fluid to flow to and through membrane 110. Members 140 and 150 are also configured to allow detection of analytes, after the analytes have been captured on membrane 110. A variety of different materials may be used for membrane 110, including, but not limited to, Nuclepore ® track-etched membranes, nitrocellulose, nylon, and cellulose acetate. Generally, the material used for membrane 110 should have resistance to non-specific binding of antibodies and stains used during the visualization and detection processes. Additionally, membrane 110 is composed of a material that is inert to a variety of reagents, buffers, and solvents. Membrane 110 may include a plurality of sub-micron pores that are fairly evenly distributed. The use of membranes having an even distribution of pores allows better control of fluid flow and control of the isolation of analytes.

Members 140 and 150 are composed of a material that is substantially transparent to wavelengths of light that are used to perform the analyte detection. For example, if the analyte detection method requires the use of ultraviolet light, member 140 should be composed of a material that is substantially transparent to ultraviolet light. Member 140 may be composed of any suitable material meeting the criteria of the detection method. A transparent material that may be used to form member 140 includes, but is not limited to, glass, quartz glass, and polymers such as acrylate polymers (e.g., polymethylmethacrylate). In some embodiments, both top member 140 and bottom member 150 are composed of transparent materials. The use of transparent materials for the top member and the bottom member allow detection to be performed through the membrane based flow sensor.

As shown in Figure 1, membrane 110 is sandwiched between top member 140 and bottom member 150. Bottom member 150 and/or top member 140 may include indentations configured to hold a membrane. For example, in Figure 1, bottom member 150 includes an indentation 152 that is configured to receive membrane 110, along with any other accompanying pieces that are used to support or seal membrane 110. Indentations or cavities may be etched into top member 140 and/or bottom member 150 using standard etching techniques.

Referring to Figure 1, bottom member 150 includes a first indentation 152, which is configured to receive a membrane support 130. Bottom member also includes a second indentation 154. Second indentation is configured such that membrane support 130 is inhibited from entering the second indentation. Second indentation may include a ridge disposed near the membrane support 130 such that membrane support 130 rests upon the ridge. Alternatively, as depicted in Figure 1, second indentation may be to may have a size that is smaller than the size of membrane support 130. In either case, when assembled, membrane support 130 is inhibited from entering second indentation 154, thus creating a cavity under membrane support 130. Cavity 154 may be used to collect fluids that pass through the membrane support 130 prior to exiting the system.

Membrane support 130 is configured to provide support to membrane 110 during use. Membrane support 130 may be formed from a porous material that allows fluid to pass through the membrane support. The pores of membrane support 130 should have a size that allows fluid to pass through membrane support 130 at a speed that is equal to or greater than the speed that fluid passes through membrane 110. In one embodiment, pores of membrane support 130 are larger than pores in membrane 110. The pores, however, cannot be too large. One function of membrane support 130 is to provide support to membrane 110. Therefore, pores in membrane support 130 should be sufficiently small enough to inhibit sagging of membrane 110 during use. Membrane support 130 may be formed of a variety of materials including, but not limited to, polymeric materials, metals, and glass. In one embodiment, a polymeric material (e.g., celcon acrylic) may serve as a material for membrane support 130. Additionally, membrane support 130 helps to keep the membrane planar during use. Keeping the membrane planar simplifies detection of the analytes by allowing the capture and detection of the analytes on a single focal plane.

Membrane 110, as described above, may rest upon membrane support 130 when the membrane based flow sensor 100 is assembled. In some embodiments, a gasket 120, may be positioned on top of membrane 110. A gasket may be used to provide a fluid resistant seal between members 130 and 140 and membrane 110. Gasket may inhibit the leakage of fluid from the system during use.

Top member 140 may include a fluid inlet 160. Fluids for analysis may be introduced into device 100 via fluid inlet 160. Fluid inlet 160 may pass through a portion of top member 140. In some embodiments, a channel 162 may be formed in top member 140 such that tubing 164 may be inserted into channel 162. Channel 162 may turn near the center of the top member to deliver the fluids to an upper surface of membrane 110.

Bottom member 150 may include a fluid outlet 170. Fluids that are introduced into the device 100 via fluid inlet 160 pass through top member 140 and through membrane 110. The fluids are then collected in cavity 154. A fluid outlet 170 may pass through a portion of bottom member 150. In some embodiments, a channel 172 may be formed in bottom member 150 such that tubing 174 may be inserted into channel 172. Channel 172 may be positioned to receive fluids that are collected in cavity 154 during use.

Optionally, a washing fluid outlet 180 may be formed in top member 140. Washing fluid outlet 180 is configured to receive fluids that pass through or over membrane 110 during a washing operation. Washing fluid outlet 180 may pass through a portion of top member 140. In some embodiments, a channel 182 may be formed in top member 140 such that tubing 184 may be inserted into channel 182. Channel 182 may be positioned to receive fluids that are used to wash membrane 110 during use.

Membrane 110 is selected from a material capable of filtering the analytes of interest from a fluid stream. For examples, if lymphocytes represent the analyte of interest, the filter should be capable of removing lymphocytes from a fluid stream. A suitable membrane may include a plurality of pores that have a size significantly less than the size of the analyte of interest.

Membranes may be formed from a variety of materials known in the art. In one embodiment, membrane 110 may be a track-etched Nuclepore^{™} polycarbonate porous membrane. A Nuclepore membrane is available from Whatman plc. Membrane 110 may be about 5-10 microns in thickness. Membrane 110 includes a plurality of pores. Pores may range from about 0.2 µm in diameter up to about 12 µm in diameter. Porous membrane filters can be supported by a plastic screen disc (Celcon®).

Where the analyte of interest is a white blood cell, e.g., a CD4 lymphocyte, disposable membrane filters are desirable for capture. For example, a polycarbonate, track-etch membranes with 3 um pores can be secured within cavities of the flow cell, creating a means for lymphocyte capture having a surface area of about 80 mm², and an internal volume of about 20 ul. Under such conditions, deformable red blood cells (of similar diameter as lymphocytes but typically numbering 1000-fold more) pass readily through the pores under suitable fluid flow conditions while white blood cells are captured and deposited into a single imaging focal plane. Separation of red cells reduces autofluorescence and improves imaging of white blood cells directly from whole blood without additional sample processing.

Figure 2 depicts an embodiment of a membrane based flow sensor disposed in housing 200. Top member 140, gasket 120, membrane 110, membrane support 130, and bottom member 150 may be assembled and placed inside housing 200. Housing 200 may encompass membrane based fluid sensor. A cap 210 may be used to retain membrane based fluid sensor within housing 200. Cap 210 may include a window to allow viewing of membrane 110. When positioned within housing 200, fluid inlet 160, fluid outlet 170 and washing fluid outlet 180 extend from housing 200 to allow easy access to the membrane based fluid sensor 100.

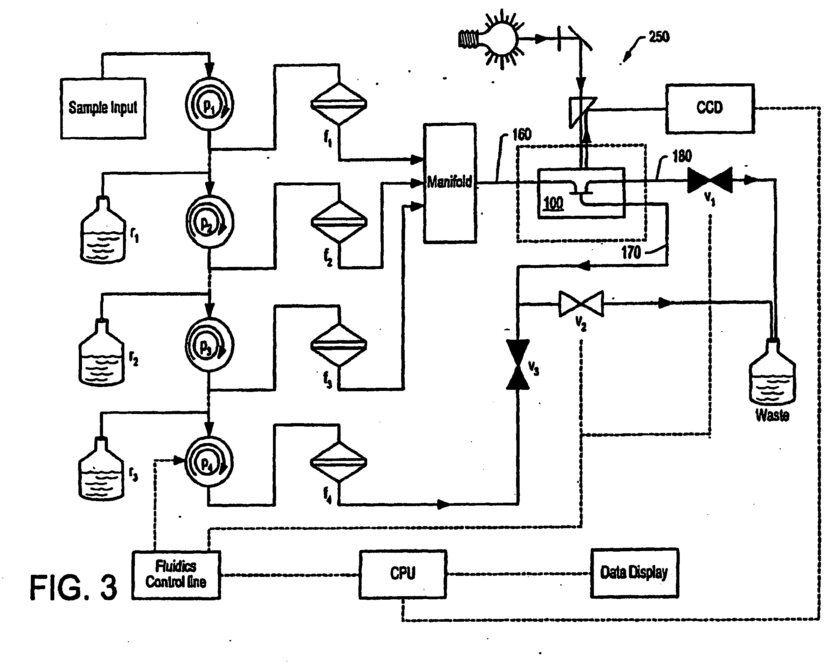

A schematic of a complete membrane based analysis system is shown in Figure 3. Analysis system includes a plurality of pumps (p₁, p₂, p₃ and p₄). Pumps are configured to deliver samples (p₁), visualization reagents (p₂ and p₃) and membrane washing fluids (p₄) to the membrane based fluid sensor 100 during use. Reagents, washing fluids, and visualization agents are passed through pre-filters (f₁, f₂, f₃, and f₄) before the fluids are sent to membrane based fluid sensor 100. Pre-filters are used to screen out large particulate matter that may clog membrane 110. The nature and pore size of each pre-filter may be optimized in order to satisfy efficient capture of large dust particles or particulate matter aggregates while resisting clogging. Pre-filter fl is configured to filter samples before the samples reach the membrane based fluid sensor 100. Pre-filter fl is configured to allow the analyte of interest to pass through while inhibiting some of the particles that are not related to the analyte of interest. For example, spores, whose size is smaller than the pores of the pre-filter f₁, are passed through the pre-filter and captured in the membrane based fluid sensor 100. After passing through pre-filters f₁ - f₄, fluids are passed through a manifold. In some embodiments, membrane based fluid sensor 100 includes a single input line. The manifold couples the different fluid lines to the single input line of the membrane based fluid sensor 100.

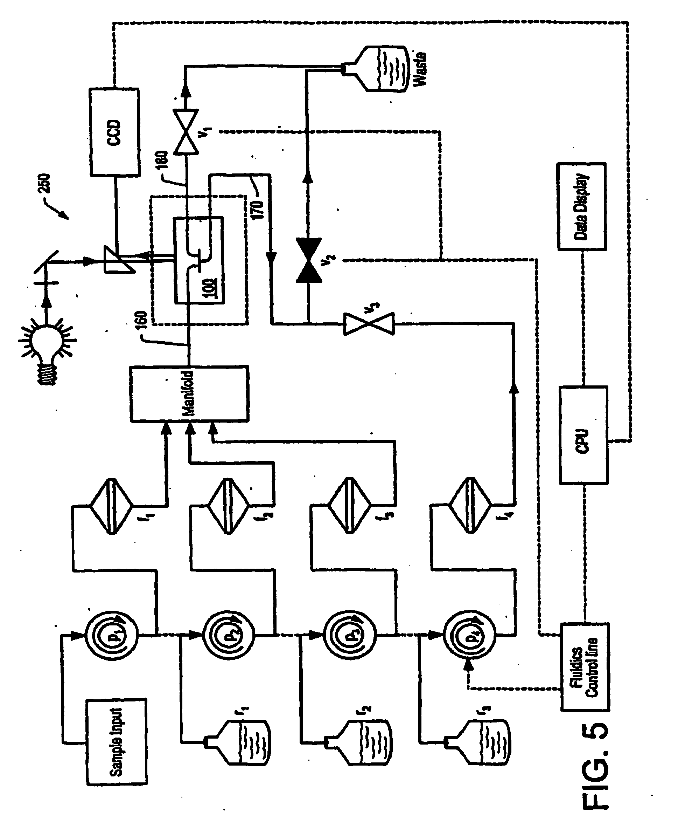

After passing through the manifold, fluids are introduced into fluid inlet of the membrane based fluid sensor 100. At appropriate times, a detector 250 is used to determine if any analytes have been captured by the membrane based fluid sensor 100. As depicted in Figure 5, a detector may be placed over a portion of membrane based fluid sensor 100 such that the detector may capture an image of the membrane. For example, detector may be placed such that images of the membrane may be taken through a window in the membrane based fluid sensor 100. Detector 250 may be used to acquire an image of the particulate matter captured on membrane 110. Image acquisition may include generating a "digital map" of the image. In an embodiment, detector 250 may include a high sensitivity sensor array (e.g., CCD or CMOS). The arrays may be interfaced with filters, light sources, fluid delivery, so as to create a functional sensor array. In one embodiment, data acquisition and handling may be performed with existing CCD or CMOS technology. In some embodiments, the light is broken down into three-color components, red, green and blue. Evaluation of the optical changes may be completed by visual inspection (e.g., with a microscope) or by use of a microprocessor ("CPU") coupled to the detector. For fluorescence measurements, a filter may be placed between detector 250 and membrane 110 to remove the excitation wavelength. The microprocessor may also be used to control pumps and valves as depicted in Figure 3. The microprocessor may also be used to control, for example, LED or other light sources, CMOS or CCD imaging components and imaging processing software.

The analyte detection system may be operated in different modes based on which valves are opened and closed. A configuration of a system in a "flow through" mode is depicted in Figure 3. In this mode, fluid is passed from the manifold to the membrane based fluid sensor 100 to allow capture of analytes or the addition of development agents. Fluids for analysis may be introduced into membrane based fluid sensor 100 via fluid inlet 160. During a "flow through" operation, valve V₁ is placed in a closed position to inhibit the flow of fluid through wash fluid outlet 180. The fluids may, therefore, be forced to pass through membrane based fluid sensor 100 exit the sensor via fluid outlet 170. Valve V₂ is placed in an open position to allow the flow of fluid to the waste receptacle. Valve V₃ is placed in a closed position to inhibit the flow of fluid into the wash fluid supply line.

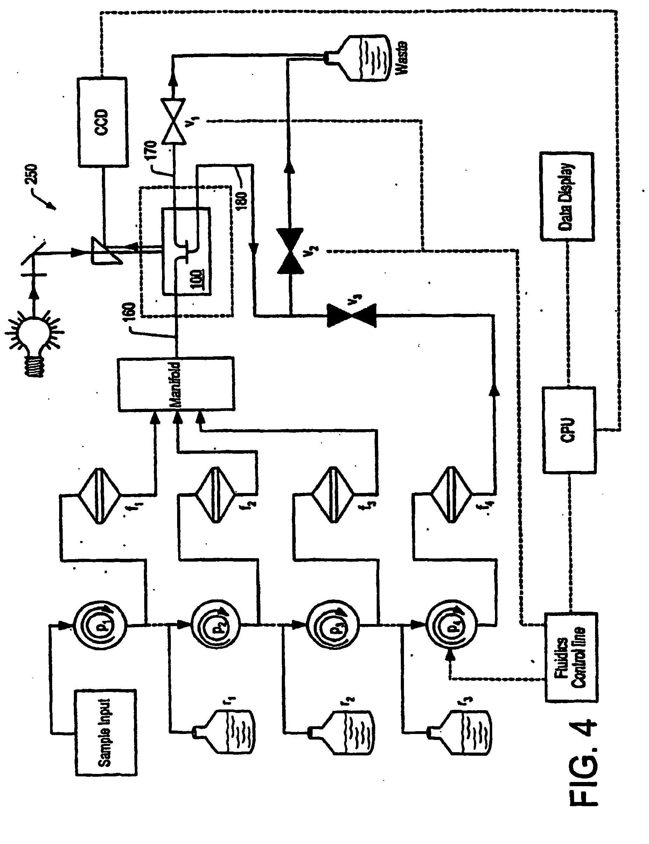

The analyte detection system may also be operated in a "lateral membrane wash" mode, as depicted in Figure 4. In this mode, the membrane is cleared by the passage of a fluid across the collection surface of the membrane. This allows the membrane to be reused for subsequent testing. Fluids for washing the membrane may be introduced into sensor 100 via fluid inlet 160. During a "lateral membrane wash" operation, outlet valves V₂ and V₃ are placed in a closed position to inhibit the flow of fluid through fluid outlet 170. The closure of outlet valves V₂ and V₃ also inhibits the flow of fluid through the membrane of sensor 100. The fluids entering sensor 100 may, therefore, be forced to exit sensor 100 through washing fluid outlet 180. Valve V₂ is placed in an open position to allow the flow of fluid through washing fluid outlet 180 and into the waster receptacle. Since fluid is inhibited from flowing through the membrane, any analytes and other particles collected by the membrane may be "washed" from the membrane to allow further use.

The analyte detection system may also be operated in a "backwash" mode, as depicted in Figure 5. During a backwash operation, fluid outlet 170 is used to introduce a fluid into the analyte detection system, while wash fluid outlet 180 is used to allow the fluid to exit the device. This "reverse" flow of fluid through the cell allows the membrane to be cleared. In an embodiment, valves may be configured as depicted Figure 5, with the washing fluid being introduced through fluid outlet 170. Specifically, valves V1 and V3 are open, while valve V2 is closed.

Either a lateral membrane wash or a back flush treatment may be used to clear analytes and other particles from a membrane. Both methods of clearing the membrane surface may be enhanced by the use of ultrasound or mechanical agitation. During use, analytes in the fluid sample are trapped by the membrane since the analytes are bigger than the openings in the membrane. The analytes tend to be randomly distributed across the membrane after use. Analytes that occupy positions on the membrane that are between the positions of pores may be more difficult to remove than analytes that are positioned on or proximate to a pore in the membrane since the force of the backwash fluid may not contact the analytes. During backwash and lateral wash operations, removal of trapped analytes may be enhanced by the use of ultrasound of mechanical agitation. Both methods cause the analytes to move across the membrane surface, increasing the chances that the analyte will encounter a column of washing fluid passing through one of the pores.

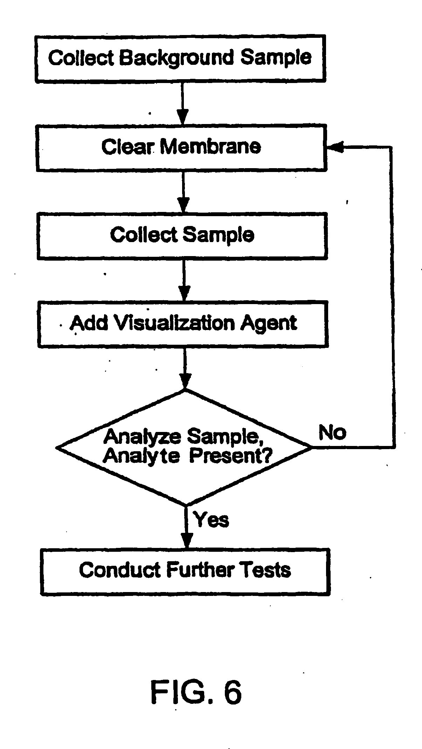

Analyte detection system may be used to determine the presence of analytes in a fluid system. One embodiment of a process for determining analytes in a fluid sample is depicted in the flow chart of Figure 6.

Prior to the analysis of any samples, a background sample may be collected and analyzed. Solid analytes are typically collected and stored in a liquid fluid. The liquid fluid that is used to prepare the samples, may be analyzed to determine if any analytes are present in the fluid. In one embodiment, a sample of the liquid fluid used to collect the solid analytes is introduced into an analyte detection device to determine the background "noise" contributed by the fluid. Any particles collected by the membrane during the background collection are viewed to determine the level of particulate matter in the liquid fluid. In some embodiments, particles collected by the membrane during the collection stage may be treated with a visualization agent to determine if any analytes are present in the liquid fluid. The information collected from the background check may be used during the analysis of collected samples to reduce false positive indications.

After collection of the background sample, the membrane may be cleared using either a back flush wash or a lateral wash, as described herein. After clearing the membrane, the system may be used to analyze samples for solid analytes

As the collected sample is passed through the porous membrane, the porous membrane traps any analytes that have a size that is greater than the size of the pores in the porous membrane. Collection of particles may be continued for a predetermined time, or until all of the collected sample has been passed through the membrane.

After collection, the analytes collected by the membrane may be analyzed using a detector. In some embodiments, the detector may be a camera that will capture an image of the membrane. For example, a detector may be a CCD or CMOS camera. Analysis of the particles captured by the membrane may be performed by analyzing the size and/or shape of the particles. By comparing the size and/or shape of the particles captured by the membrane to the size and shape of known particles the presence of a predetermined analyte may be indicated. Alternatively, analytes will react to a variety of visualization agents (e.g., colored and fluorescent dyes). Analytes captured by the membrane are analyzed using an appropriate detector. The presence of particles that have the appropriate color and/or fluorescence may indicate the presence of the analyte being tested for.

In one embodiment, detection of CD3, CD4 and CD8 cells in whole blood is desired. Prior to delivery to the flow cell, whole blood sample can be incubated with fluorophore-conjugated anti-CD3 and anti-CD4 antibodies for suitable duration (e.g., 8 minutes). Pre-incubation can enhance detection. Delivery of test samples to the flow cell can be regulated through the use of a fluidics controller (e.g., peristaltic pump).

Digital images from one region of the lymphocyte capture membrane can obtained with two different emission filters, e.g., one specific for an Alexa488-conjugated antibody (recognizing CD4+ T lymphocytes and providing green signal) and the other specific for the Alexa647-conjugated antibody (recognizing CD3+ T lymphocytes and providing red signal). Automated digital merging of the two images then distinguishes the CD3+/CD4+ T lymphocytes of interest (i.e., CD4 cells which appear yellow), from the CD4+/CD3- monocytes (which appear green), and the CD3+/CD4- T lymphocytes (which appear red).

The analysis of the particles may indicate that an analyte of interest is present in the sample. In this case, the particles may be flushed from the membrane and sent out of the system for further testing. Further testing may include techniques such as cultures or ELISA techniques that may allow more accurate determination of the specific analytes present. Alternatively, the particles may be sent to a sensor array, as described herein, for further testing. If no significant amounts of analytes are found on the membrane, the membrane may be washed and other samples analyzed.

User-defined threshold criteria may be established to indicate a probability that one or more specific analytes are present on the membrane. The criteria may be based on one or more of a variety of characteristics of the image. In some embodiments, the criteria may be based on pixel or color fingerprints established in advance for specific analytes. The characteristics that may be used include, but are not limited to, the size, shape, or color of portions of matter on the image, the aggregate area represented by the matter, or the total fluorescent intensity of the matter.

The membrane system may include a computer system (not shown). Computer system may include one or more software applications executable to process a digital map of the image generated using detector. For example, a software application available on the computer system may be used to compare the test image to a pre-defined optical fingerprint. Alternatively, a software application available on computer system may be used to determine if a count exceeds a pre-defined threshold limit.

A detector may be used to acquire an image of the analytes and other particulate matter captured on a membrane. The image acquired by the detector may be analyzed based on a pre-established criteria. A positive result may indicate the presence of a microbe. The test criteria may be based on a variety of characteristics of the image, including, but not limited to, the size, shape, aspect ratio, or color of a portion or portions of the image. Applying test criteria may allow analytes of interest to be distinguished from background particulate matter. During analysis, the flow of sample through from a fluid delivery system may be continued.

In some embodiments, a positive result may create a presumption that the fluid contains a particular analyte. If the image yields a positive result with respect to the test criteria, a sample of the fluid may be subjected to a confirmatory or specific testing. On the other hand, if the image yields a negative result with respect to the test criteria, membrane may be rinsed and the preceding method may be carried out for fluid from another sample.

During analyte testing a sample may be introduced into the analyte detection device. A trigger parameter may be measured to determine when to introduce the visualization agent into the analyte detection device. Measurement of the trigger parameter may be continuous or may be initiated by a user. Alternatively, the stain may be introduced into the analyte detection device immediately after the sample is introduced.

In one embodiment, the trigger parameter may be the time elapsed since initiation of introducing the fluid into an analyte detection device at a controlled flow rate. For example, the stain may be introduced 20 seconds after initiation of introducing the fluid sample into an analyte detection device at a flow rate of 1 milliliter per minute. In another embodiment, the trigger parameter may be the pressure drop across the membrane. The pressure drop across the membrane may be determined using a pressure transducer located on either side of the membrane.

In another embodiment, the trigger parameter may be the autofluorescence of analytes captured by the membrane. A detector may be switched on until a pre-defined level of signal from the autofluorescence of the analytes has been reached. In still another embodiment, filtering software may be used to create a data map of the autofluorescence of the matter on the membrane that excludes any pixels that contain color in a blue or red spectral range. The data map may be used to compute a value for particles that are autofluorescent only in the "pure green" portion of the visible spectrum.

In some embodiments, a presumptive positive result may be inferred if the trigger parameter exceeds a certain value without applying a stain. For example, a presumptive positive result may be inferred where the autofluorescence value is more than twice the value that would indicate application of a stain. In such a case, the application of a stain may be dispensed with and a confirmatory test may be conducted for the sample.

If the value of the trigger parameter is less than would indicate proceeding directly to the confirmatory test, but exceeds the value established to trigger the application of a stain, then a stain may be introduced into an analyte detection device.

Collecting a sample of a fluid may include gathering a sample from a solid, liquid, or gas. In some embodiments, the sample may be derived from collecting air from a target environment in an aerosol form, then converting aerosol into a hydrosol. For example, particles from 500 liters of an air sample may be collected deposited into about 0.5 milliliters of liquid. U.S. Patent No. 6,217,636 to McFarland, entitled "TRANSPIRATED WALL AEROSOL COLLECTION SYSTEM AND METHOD," which is incorporated herein by reference as if fully set forth herein, describes a system for collecting particulate matter from a gas flow into a liquid using a porous wall.

The following examples are provided as a further description of the invention, and to illustrate but not limit the invention.

### EXAMPLES

### Example 1

### Microchip-based Assay for Measurement and Quantification of CD4+ T Cells

### Design and Development of Flow Cell Analysis Chamber

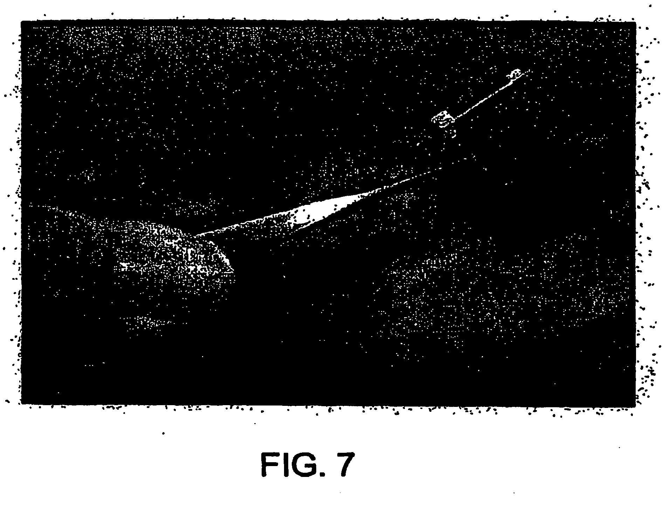

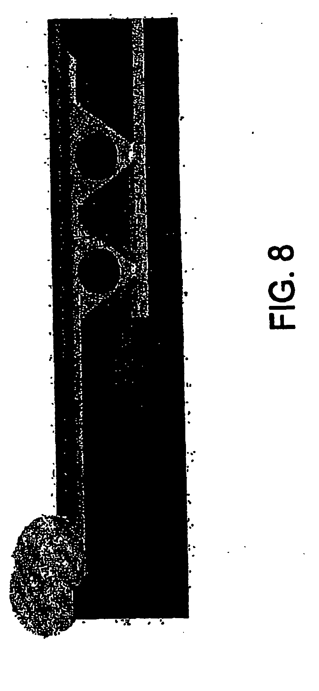

A chip-based sensor array composed of individually addressable microwells on a single silicon or plastic microchip has been developed. Microwells were created using molded-plastic methods, optimized, and the resultant structures, made with an anisotropic etch, served as miniaturized reaction vessels and analysis chambers. Each microwell has a volume of approximately 30 nanoliters (nL); a single microliter (µL) of fluid provides sufficient sample to complete multiple assays. Microwells possess pyramidal pit shapes, with openings that allow for both fluid flows through the analysis chambers as well as optical analysis of reactions occurring on the floor of the microwell, or on microbeads or a membrane filter resting within the microwell. Current chips contain up to 100 microwells on a single, dime-sized chip (Figures 7 and 8).

The microchip is anchored inside the microfluidic flow cell. The modified version of the flow cell is enclosed within a three-piece stainless steel casing that includes a flat platform, permanently affixed to a circular vertical support, which is in turn connected to a convertible (screw-on) cap (Figure 9). Within the metal casing there are top and bottom circular polymethylmethacrylate (PMMA) inserts. These inserts allow, through integrated stainless steel tubing (Microgroup, Medway, MA), the introduction (top left inlet) and drainage (top right and bottom outlets) of fluids delivered to the flow cell via the fluid delivery system (Figure 9). In addition to being equipped with a drain, the bottom PMMA insert also features a plastic screen disc that acts as a support for the lymphocyte capture membrane, a 3.0m microporous filter (Nuclepore^{®}, Whatman, Clifton, NJ). A gasket between the membrane and the top insert prevents leaks and ensures that the entire sample is delivered into the flow cell and filtered through the membrane. The top outlet is utilized in conjunction with lateral fluid flow for the removal of air bubbles, as necessary.

### Fluid Delivery System

In initial studies, a single peristaltic pump was used to deliver both the sample and wash buffers to the flow cell. In subsequent designs, a partially automated fluid delivery system was used. This version uses a platform equipped with two miniature OEM peristaltic pumps, each in conjunction with a pinch valve (v₁ and v₂, respectively) utilizing .031" silicone tubing and capable of delivering flow rates of 0.046-0.92 mls/minute to the flow cell. Integrated software directs delivery of pre-stained blood sample and wash cycles using the appropriate combination of pumps and valves. Sample and wash fluid that filter through the capture membrane, including red blood cells, are captured in a waste reservoir. A third valve (v₃) positioned after the top flow cell outlet is used in conjunction with p₂ to remove any air bubbles formed during the assay.

For optimal performance of the microfluidics of the flow cell, and for retention of lymphocytes and removal of red blood cells, 3.0-micron capture membranes were used in subsequent studies. These membranes effectively filtered red blood cells without loss of retained lymphocytes. Optical Station and Image Capture and Analysis

The analysis flow cell was positioned on the stage of a modified compound BX2 Olympus microscope equipped with a 4X objective lens and a high-pressure mercury lamp as a light source. Focusing was maintained on a fixed plane on the membrane throughout the duration of the assay. Visualization of Alexa647-stained (red) lymphocytes was achieved using a Cy5^{™} filter cube (620 nm excitation, 660 long pass beam splitter dichroic mirror, and 700 nm emission, Olympus), while Alexa488-stained (green) lymphocytes were visualized with a fluoroisothiocyanate (FITC) filter cube (480 nm excitation, 505 long pass beam splitter dichroic mirror, and 535 ± 25 nm emission, Olympus). A motorized stage allowed for multiple images to be taken at different areas of the membrane.

For each study subject, a total of five non-overlapping regions of the lymphocyte capture membrane in the flow cell were imaged using a 12-bit Digital Video Camera (DVC) 1312C (DVC, Austin, TX) charge-coupled device (CCD) mounted on the microscope. Each region was imaged twice, once using the Cy5^{™} filter cube for detection of Alexa-488 fluorescence, and once using the FITC filter cube for detection of Alexa-647 fluorescence. Except for the dose-response studies, these two images were merged to produce a single digital image prior to analysis.

Images were analyzed using a custom algorithm developed in a commercial image processing software package (Image-Pro Plus, Media Cybernetics). In this algorithm, thresholds for red, green and blue intensity were established for optimal definition of lymphocytes against background fluorescence; lymphocytes were also characterized by their geometry (size and shape). Background correction was performed to remove autofluorescence, and objects identified as presumptive cells were enhanced by standard image processing protocols to blur sharp edges. Cells thus identified were then counted in an automated fashion, with results recorded in a spreadsheet (Microsoft Excel, Redmond, WA) as numbers of CD4+CD3-, CD4+CD3+, CD8+CD3-, CD8+CD3+ and CD4+CD8+ cells, depending on the combination of antibodies used.

### CD4+ Lymphocyte Detection

As described above, the microchip system with a 3.0 µm porous filter was adapted for optimal lymphocyte capture in order to establish the basis for a microchip CD4+ count. These studies were performed with peripheral blood mononuclear cells (PBMCs) isolated from healthy volunteers. For these experiments, buffy coats (lymphocyte fraction of whole blood) were obtained and PBMCs isolated by Ficoll density gradient centrifugation. T lymphocyte subsets were obtained to >98% purity from PBMCs by immunomagnetic separation (Miltenyi Biotec, Auburn, CA) erythrocyte rosetting (Stem Cell Technologies). The subsets were resuspended at a stock concentration of 10⁶ cells/mL in RPMI, and used within 24 hours of preparation. Alternatively, cells were cryopreserved in 10% dimethylsulfoxide (DMSO) in RPMI and thawed immediately prior to use. In all cases, cells were counted and assessed for viability immediately before use.

Purified CD4 cells were labeled with Alexa488-conjugated anti-CD4 antibody (Molecular Probes, Eugene, OR) at room temperature for 5 minutes and then introduced into the flow chamber at controlled rate of 0.3 mL/minute at varying dilutions (ranging from 0 to 200,000 CD4+ cells/mL). Samples were injected into the flow chamber manually or via an automated process using a fast pressure liquid chromatography system (Pharmacia) driven by Unicorn 3.0 software (Amersham).

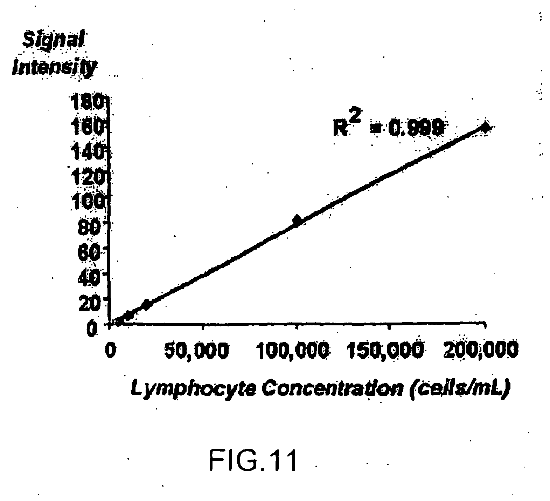

After sample introduction, the chamber was washed with 2 to 5mL of phosphate buffered saline (PBS), introduced at 1.2 mol/minute. This volume represents 10,000 dead volumes, and removes unwanted components of blood, including platelets and erythrocytes, and also significantly reduces background signal. The chamber was then imaged using fluorescence microscopy. Raw images were obtained for increasing numbers of CD4 cells, ranging from 0 to 200,000 cells per mL, corresponding to the physiologic ranges seen in patients with advanced AIDS. As shown in Figure 11, there is a linear correlation between the number of cells in the sample and the light intensity when measured from a digital image by pixel analysis (R²=0.999). This established that the microchamber and digital image analysis system could be used to accurately measure and detect populations of lymphocytes labeled with fluorescent markers.

### Example 2

### Measurement of CD4:Q8 Ratios and CD4+ Percentages from Whole Blood

A rapid, whole blood assay (without red blood cell lysis, extra buffers or additional sample processing) for detecting CD4+ and CD8+ cells and establishing CD4:CD8 ratios was developed, based on the microchip discussed in Example 1. Adjustment of the flow parameters and dilution of the sample revealed an optimal flow rate of 0.8 mL/minute and a dilution of whole blood of 1:20.

The blood was obtained by venipuncture from healthy volunteers or HIV-infected subjects at the Massachusetts General Hospital and the Botswana-Harvard Laboratory in Gabarone, Bostwana. For the measurement of CD4:CD8 ratios and CD4+percentages, 33 microliters of whole blood was mixed with 3 microliters of fluorophore-conjugated antibodies to CD3, CD4 or CD8 for staining. An optimized staining protocol was established for each of the antibodies utilized in this study by performing tube reactions and on-slide observation of the results. Antibodies were microfuged (at 3,000 x g for 2 minutes at room temperature) prior to use and only the supernatant reagent was used for staining. This process ensured removal of any fluorescent particulate matter that could potentially be captured by the membrane and thus interfere with imaging. Stained blood samples were brought up to 500 µl with PBS, introduced directly into the flow cell and subsequently washed with 2 ml of PBS. Images of labeled cells captured on the membrane were then obtained and analyzed as described above. In a subset of samples, cells that were not retained on the filter were collected from the waste reservoir and counted, to determine the efficiency of capture. For some experiments, after image capture a fixative (2% paraformaldehyde / 2.5% glutaraldehyde) was introduced into the flow cell, followed by a rinse with PBS. The filter was removed from the flow cell, fixed for 90 seconds with OsO₄ vapor, and then dehydrated with EtOH/HMDS for scanning electron microscopy (SEM).

The total time from blood collection to image acquisition, analysis and results was under 15 minutes. Initial studies of fluorophores revealed that photostability and pH were important determinants of the fluorescent signal in the system. Hence, although a variety of fluorophore-antibody combinations were evaluated in pilot studies, for all subsequent studies only the Alexa class of fluorophores was used. The availability of only Alexa-488 and Alexa-647 conjugated antibodies against CD antigens limited the system to two-color imaging. A series of raw images were collected from HIV-infected subjects using the whole blood assay. Alexa488-conjugated antibodies label the CD4+ cells green. All T lymphocytes stain red with the Alexa647-conjugated antibody to CD3. Automatic merging of the images allows the system to distinguish the CD3+CD4+ T lymphocytes of interest, which appear yellow, from the CD4+CD3- monocytes (green), and CD3+CD4- T lymphocytes (red). Automated masking of the unwanted cells leaves only the CD4+CD3+ cells of interested to be counted.

Using a custom algorithm developed within the image processing software environment of the system, the total number of each subtype of lymphocyte was automatically calculated from the raw images and sent to a data file.

In this algorithm, thresholds for red, green and blue intensity were established for optimal definition of lymphocytes against background fluorescence; lymphocytes were also characterized by their geometry (size and shape). Background correction was performed to remove autofluorescence, and objects identified as presumptive cells were enhanced by standard image processing protocols to blur sharp edges. Cells thus identified were then counted in an automated fashion, with results recorded in a spreadsheet (Microsoft Excel, Redmond, WA) as numbers of CD4+CD3-, CD4+CD3+, CD8+CD3-, CD8+CD3+ and CD4+CD8+ cells, depending on the combination of antibodies used.

For each study subject, five non-overlapping images were obtained and used for cell counting, increasing the size of the sample cell population and improving accuracy.

### Correlation with Flow Cytometry.

Results for CD4 expressed as percentage of total T lymphocytes (number of CD4+CD3+ cells/number of total CD3+ cells) and CD4:CD8 ratios (CD4 percentage/CDS percentage) obtained from the microchip system were compared with results obtained in parallel by flow cytometry for each of the study subjects, as shown in Figure 12.

Agreement between the two methods was excellent across the range of absolute CD4 counts, CD4 percentages of total T lymphocytes, and CD4/CD8 ratios seen in HIV infection. For absolute CD4 counts, a methods comparison according to the approach of Passing and Bablok23 showed an excellent correlation with flow cytometry (Figure 12A), and a Bland-Altman plot24 showed zero bias and good 95% limits of agreement (Figure 12B). The calculated Pearson correlation coefficient is r=0.92. Similar results were obtained for both CD4 counts reported as a percentage of total CD3+ T lymphocytes (Figure 12C-D), and CD4:CD8 ratios (Figure 12E-F). The limits of agreement were tighter for the CD4 percentage and CD4:CD8 ratio assessments than for absolute CD4 counts. This likely reflects expected variations in volumetric delivery of sample in the prototype, which affects absolute counting but not ratios. In addition, because there was a significant delay in processing some samples for flow cytometry but not the microchip assay, some microchip results may in fact be closer to the "true" value than flow cytometry results. Close agreement between the two methods is also seen in the correlation for the subset of 15 subjects with absolute CD4 counts below 200 cells/mL.

### Example 3

### Microbead Immunoassay

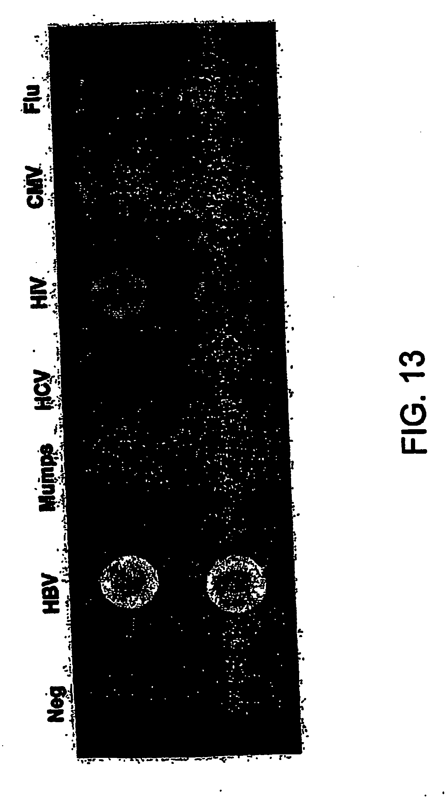

Agarose microbeads were coated with antigens specific to a variety of pathogens, including HIV gp41/gp120, HIVp24 and hepatitis B surface antigen. Beads coated with antibody to specific antigens were placed in the wells of a silicon microchip and serum samples containing known titers of antibodies were then run through the microchip system, and detected by fluorescence microscopy using Cy2-labelled secondary antibodies. Figure 13 shows results of one such experiment; with serum containing antibodies against HIV and hepatitis B easily detected using the microchip approach.

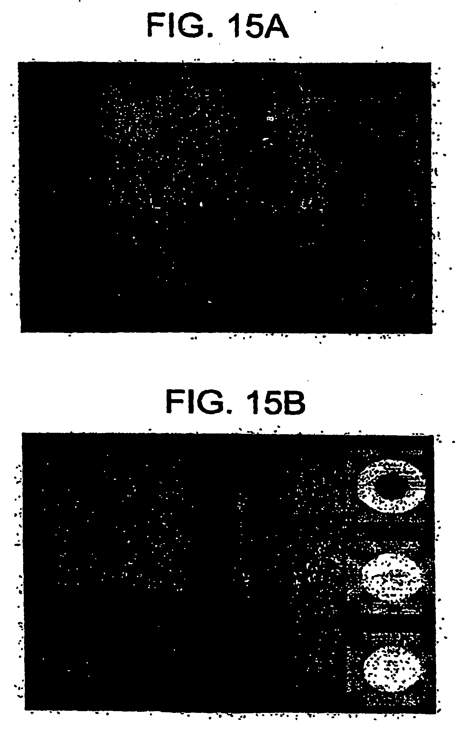

Figure 15 shows the results of another microbead experiment where the HIV p24 antigen was detected in human serum containing 100 pg/ml of HIV p24 antigen. The same principle can be applied to any immunoassay, including liver enzymes.

### Example 4

### Measurement and Quantification of RNA From the Fingerstick Samples of Whole Blood

One way to show that anti retroviral drugs are working to control the clinical progression of AIDS is through monitoring viral load before and after treatment. Viral load measurements often help physicians to decide when to start administering the antiretroviral treatments. For example, when CD4 counts are on the borderline (350 cells/mm³), a high viral load might be taken as a reason to begin treatment with antiretroviral treatments and a low viral load might be taken as a reason to wait. In addition, viral load measurements guide physicians in designing an appropriate course of treatment.

At present, HIV viral load is commonly assayed by PCR-based methods that involve viral sequence amplification using a thermocycler, followed by detection using one of several real-time fluorescence detection methods. This assay nevertheless requires reliable electricity and highly skilled laboratory personnel to perform the additional processing. These requirements significantly limit its use in resource-poor settings, where the needs for affordable HIV laboratory tests are tremendous. The assays presented herein describe the development of sample, inexpensive, electricity-independent, microchip-containing assay for HIV RNA. Assay Parameters

For these assays, a microchip-based sensor assay was created; composed of individually addressable agarose microbeads coupled to biotinylated molecular beacon DNA capture probes. Probes were selectively arranged in micromachined cavities localized on silicon microchip wafers. Samples containing target DNA and RNA flow through these microchambers and bind to the capture sequence, releasing a fluorescent signal. To establish proof-of principle microchip-based HIV RNA essays, HIV-specific molecular beacons (clade B gag, *tgcagaatgggatagattg*) were attached to microbeads, and samples containing complementary HIV RNA sequences at concentrations ranging from femtomolar to nanomolar were introduced to the microchip. Annealing of HIV RNA in the sample, with the capture sequence attached to the microchip, opens up the molecular beacon, leading to bright fluorescence. As shown in Figure 15A the baseline signal (no HIV RNA) does not lead to an opening of the molecular beacon and, therefore, shows only minimal fluorescence signal. In contrast, as shown in Figure 15B, addition of sample containing 100 pM HIV RNA results in a bright fluorescence image.

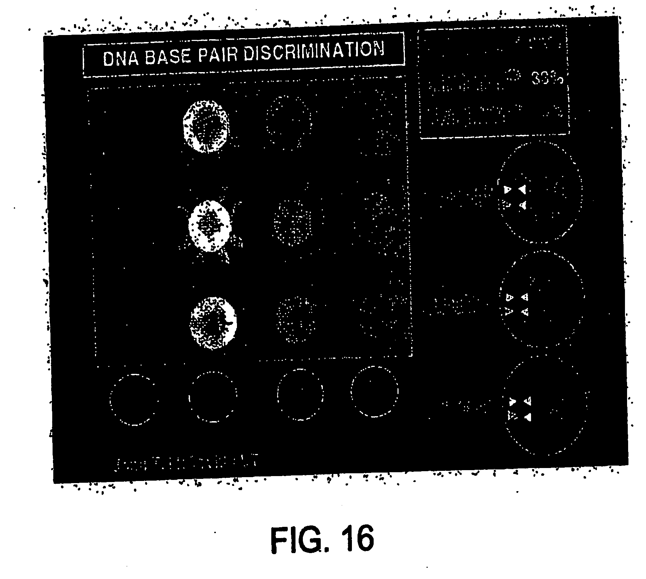

To establish specificity of the HIV RNA assay, the experiment was performed wherein beads were coated with 18 base pair DNA oligomers differing from each other by a single nucleotide, complementary sequences for each of the target probes were labeled with distinct fluorochromes, and mixed in a 1:1:1 ratio in solution. Chips were then tested against this equimolar solution and binding was detected by fluorescence microscopy. As shown in Figure 16, fluorochrome-labeled sequences bound only to their complimentary target probes, and not to target probes mismatched by a single nucleotide.

For the above described microchip based assay, optimal binding of target RNA to the molecular beacon, and a threshold of detection were established. Unlike other approaches, the assays do not require 1) direct isolation of DNA from an AIDS patient followed by 2) sequence amplification using a thermocycler (e.g., in *vitro* transcription to generate RNA for the analysis).

### Example 5

### Microchip-Based Assay for Measurement and Quantification of Liver Enzymes From the Fingerstick Samples of Whole Blood

Mild to moderate elevations in two liver enzymes, alanine transamine (ALT) and aspartamine transamine (AST), are related to liver failure, which remains the most common cause of death in people with AIDS. Liver injury is a major limiting factor in the effectiveness of current HIV treatment, and emphasize the importance of monitoring ALT and AST in AIDS patients.

Present serological assays used for monitoring ALT and AST are not suitable for use in resource-poor settings due to high cost, requirements of reliable electricity and highly skilled workforce. The results presented herein overcome the above-described limitations by describing inexpensive, electricity-independent, microchip-based essay for measuring the levels of ALT and AST.

### Assay Parameters

For assays of liver enzymes, antibodies specific for alanine aminotransferase (ALT) and aspartate aminotransferase (AST) can be obtained and bound to microbeads. The samples containing known quantities of ALT and AST can be introduced to the chip to evaluate the sensitivity and detection threshold of the microchip assay. After the sample introduction, the chamber is washed and imaged under fluorescence microscopy. For the fluorescence detection, a second sandwich antibody labeled with a fluorophore is introduced. Total fluorescence intensity is then correlated against standard ALT and AST concentrations, and the basic performance characteristics (linear range, detection threshold, reproducibility and validity) can be assessed.

## Claims

1. A method for detecting an HIV-associated analyte in a sample of blood comprising:
a) passing an HIV-associated analyte coupled to a fluorescence-emitting conjugate through a flow cell (100) comprising one or more cavities, wherein one or more of the cavities contains an agarose bead comprising a suitable biological agent having binding affinity for the analyte, such that the analyte is immobilized on the bead, thereby separating the analyte from the blood, wherein the analyte is alanine aminotransferase or aspartate aminotransferase;
b) delivering light to the analyte at a wavelength suitable for excitation of the conjugate;
c) digitally imaging a conjugate emitted fluorescent signal using a detector (250).

2. The method of claim 1, wherein the biological agent is an antibody.

3. The method of claim 1 or claim 2, wherein the blood is obtained from a fingerstick sample of blood.

4. The method of any one of claims 1 to 3 wherein one or more of the cavities contains a filter formed by a polycarbonate porous membrane.

5. The method of any one of claims 1 to 4, wherein the detector is a CCD digital camera.

6. The method of any one of claims 1 to 5, wherein the detector is a CMOS detector.

## Patentansprüche

1. Verfahren zum Nachweis eines HIV-assoziierten Analyten in einer Blutprobe, das Folgendes umfasst:
a) Passieren eines an ein Fluoreszenz-emittierendes Konjugat gekoppelten HIV-assoziierten Analyten durch eine Durchflusszelle (100), die eine oder mehr Kavität(en) umfasst, worin eine oder mehr der Kavitäten ein Agarose-Bead enthält/enthalten, umfassend ein geeignetes biologisches Agens mit Bindungsaffinität zum Analyten dergestalt, dass der Analyt auf dem Bead immobilisiert wird, wodurch der Analyt vom Blut getrennt wird, worin der Analyt für Alanin-Aminotransferase oder Aspartat-Aminotransferase steht;
b) Aussenden von Licht an den Analyten bei einer Wellenlänge, die für die Anlegung des Konjugats geeignet ist;
c) digitale Abbildung eines vom Konjugat emittierten Fluoreszenzsignals mittels eines Detektors (250).

2. Verfahren nach Anspruch 1, worin das biologische Agens einen Antikörper darstellt.

3. Verfahren nach Anspruch 1 oder Anspruch 2, worin das Blut mittels Blutentnahme aus der Fingerkuppe erhalten wird.

4. Verfahren nach einem der Ansprüche 1 bis 3, worin eine oder mehr der Kavitäten ein Filter enthält/enthalten, das durch eine poröse Polycarbonatmembran gebildet ist.

5. Verfahren nach einem der Ansprüche 1 bis 4, worin der Detektor eine CCD-Digitalkamera darstellt.

6. Verfahren nach einem der Ansprüche 1 bis 5, worin der Detektor einen CMOS-Detektor darstellt.

## Revendications

1. Méthode de détection d'un analyte associé au VIH dans un échantillon de sang, comprenant les étapes consistant à :
a) faire passer un analyste associé au VIH couplé à un conjugué émetteur de fluorescence à travers une cellule; d'écoulement (100) comprenant une ou plusieurs cavités, où une ou plusieurs des cavités contiennent une bille d'agarose comprenant un agent biologique convenable ayant une affinité de fixation vis-à-vis de l'analyte, de sorte que l'analyte soit immobilisé sur la bille, séparant ainsi l'analyte à partir du sang, ou l'analyte est l'alanine aminotransférase ou l'aspartate aminotransférase ;
b) apporter de la lumière à l'analyte à une longueur d'onde convenable pour l'excitation du conjugué ;
c) réaliser l'imagerie numérique d'un signal fluorescent émis par le conjugué à l'aide d'un détecteur (250).

2. Méthode selon la revendication 1, dans laquelle l'agent biologique est un anticorps.

3. Méthode selon la revendication 1 ou la revendication 2, dans laquelle le sang est obtenu à partir d'un échantillon de sang du bout du doigt.

4. Méthode selon l'une quelconque des revendications 1 à 3, dans laquelle une ou plusieurs des cavités contiennent un filtre formé par une membrane poreuse en polycarbonate.

5. Méthode selon l'une quelconque des revendications 1 à 4, dans laquelle le détecteur est une caméra numérique DTC.

6. Méthode selon l'une quelconque des revendications 1 à 5, dans laquelle le détecteur est un détecteur CMOS.
